# EUROPÄISCHE PATENTSCHRIFT

(11) **EP 0 732 583 B1**
(45) Veröffentlichungstag und Bekanntmachung des Hinweises auf die Patenterteilung: **14.05.2003**
(21) Anmeldenummer: 96890048.0
(22) Anmeldetag: 15.03.1996
(51) Int. Cl.: G01N 21/64, G01N 33/543, G01N 33/487

(54) **Optochemischer Fluoreszenzsensor sowie ein Verfahren zur Messung der Konzentration zumindest eines Analyten in einer Probe**
Optochemical fluorescence sensor and process of measuring the concentration of at least one analyte in a sample
Capteur de fluorescence optochimique et procédé de mesure de la concentration d'au moins un analyte dans un échantillon

(30) Priorität: 17.03.1995 AT 47495
(43) Veröffentlichungstag der Anmeldung: 18.09.1996
(73) Patentinhaber: SCHALKHAMMER, Thomas, Mag.Dr., 3072 Kasten (AT); PITTNER, Fritz, Ass.Prof.Doz.Dr., 1235 Wien (AT); Leitner, Alfred Dr., 8010 Graz (AT); Aussenegg, Franz Dr., 8020 Graz (AT); Brunner, Harald Dipl.-Ing., 8045 Graz (AT); Bauer, Georg, 1170 Wien (AT)
(72) Erfinder: Schalkhammer, Thomas, Mag. Dr., 3072 Kasten (AT); Pittner, Fritz, Dr., 1235 Wien (AT); Leitner, Alfred, Dr., 8010 Graz (AT); Aussenegg, Franz, Dr., 8020 Graz (AT); Brunner, Harald, Dipl.-Ing., 8045 Graz (AT)
(74) Vertreter: Babeluk, Michael, Dipl.-Ing. Mag.

(56) Entgegenhaltungen:
- WO-A-89/09408
- CA-A- 2 076 709
- US-A- 5 017 009

## Beschreibung

Die Erfindung betrifft einen optochemischen Fluoreszenzsensor zur Messung der Konzentration zumindest eines Analyten in einer Probe, mit zumindest einer auf ein Substrat aufgetragenen Inselschicht, deren Inseln aus elektrisch leitendem Material bestehen und einen Durchmesser kleiner 300 nm aufweisen sowie ein Verfahren zur Messung der Konzentration zumindest eines Analyten in einer Probe.

Optochemische Sensoren basieren darauf, dass eine chemische Reaktion zwischen dem Sensormaterial und dem Analyten zu einer Veränderung der optischen Eigenschaften des Sensors führt. Eine Veränderung der optischen Eigenschaften kann beispielsweise in einer Änderung der Absorptions- bzw. der Fluoreszenzeigenschaften liegen, so dass die Reaktion in der Folge durch spektroskopische Methoden nachweisbar wird.

Optochemische Sensoren zur Messung chemischer Stoffkonzentrationen gewinnen zunehmend an Interesse, da sie gegenüber herkömmlichen Messeinrichtungen wesentlich kürzere Ansprechzeit, eine größere mechanische Robustheit und eine Unempfindlichkeit gegenüber elektromagnetischen Einstreuungen sowie weitere Vorteile aufweisen. Wesentlich für derartige optochemische Sensoren ist allerdings, dass das Sensormaterial dem Angriff des Analyten entsprechend ausgesetzt ist, um eine kurze Ansprechzeit zu gewährleisten.

Aus der GB-A 2 243 683 ist ein optochemischer Sensor bekannt, welcher am Ende einer Fiberoptik eine biorekognitive Schicht aufweist, die mit einem Analyten einer Probe in Kontakt treten kann. Die biorekognitive Schicht weist an Antikörpern gebundene fluoreszenzmarkierte Antigene auf, welche bei Probenkontakt durch den Analyten ersetzt werden. Die abnehmende Fluoreszenz wird als Maß für die Analytkonzentration detektiert.

Aus der DE-A1 42 10 970 ist ein Verfahren zur optischen qualitativen und quantitativen Erfassung von Biomolekülen, toxischen Substanzen, Polymeren und pharmazeutischen Wirkstoffen mittels Laserspektroskopie bekannt. Dabei werden Fluoreszenzfarbstoffe an die zu messenden Moleküle gekoppelt und über die vom markierten Molekül unbeeinflusste Fluoreszenzabklingzeit detektiert. Als Fluoreszenzfarbstoffe finden unter anderem Fluoresceine und Rhodamine Verwendung.

Aus der US 5,449,918 A ist ein optischer Sensor zur direkten und kontinuierlichen Detektion organischer Spezies in Prozessströmen bekannt, welcher auf einem Substrat eine Schicht aus Metallinseln aufweist, deren Ausdehnung im Vergleich zu sichtbarem Licht klein ist. Über der Inselschicht befindet sich eine chemisch selektive Schicht mit einer Schichtdicke <200 nm, welche einen Fluorophor enthält. Der Sensor nützt den Effekt der "Surface Plasmon Resonance" um die Fluoreszenzemission der chemisch selektiven Schicht zu verstärken. Die selektive Schicht kann als Lang-muir-Blodgett-Film auf die dünne Metallinselschicht aufgebracht werden. Die chemisch selektive Schicht enthält somit den Fluorophor gemäß einer ersten Ausführungsvariante bzw. in einer weiteren Variante einen Fluorophorfilm, welcher direkt auf der Inselschicht angeordnet ist.

Aus der WO 89/09408 A ist es bekannt ein Biomolekül, z.B. das Human Chorionic Gonadotrophin (hCG) zu detektieren, wobei ein fluoreszenzmarkierter Antikörper mit dem Analyten (hCG) und einem zweiten ebenfalls fluoreszenzmarkierten Antikörper einen Sandwich-Komplex bildet, welcher an einen Lichtleiter gebunden ist. Das beschriebene Verfahren basiert, auf dem "Resonance Energy Transfer" (bzw. Förster Transfer), wobei der Energietransfer von Fluorophor zu Fluorophor genützt wird. Die Reichweite des Resonance Energy Transfer ist allerdings auf wenige nm begrenzt.

Aufgabe der Erfindung ist es, einen optochemischen Sensor der eingangs genannten Art zu schaffen, mit welchem insbesondere Stoffkonzentrationen, von beispielsweise Antikörpern oder Enzymen, in besonders einfacher und reproduzierbarer Weise erfasst werden können, wobei auf die Verwendung von Elektroden verzichtet werden kann, und das Messergebnis auch bei überaus geringen Änderungen der zu messenden Stoffkonzentrationen mit hoher Präzision und kurzer Messzeit deutlich ablesbar ist.

Diese Aufgabe wird erfindungsgemäß dadurch gelöst, dass der Sensor eine biorekognitive Schicht, bestehend aus Proteinen, Lipiden, Nukleinsäuren oder artifiziellen Liganden aufweist, welche mittels einer Spacerschicht an der Inselschicht angeordnet ist, dass ein der Probe zusetzbares oder im Sensor vorliegendes analytspeziflsches, fluoreszierendes System vorgesehen ist, dass die biorekognitive Schicht den zu messenden Analyten direkt oder mittels analytbindender Moleküle zu binden vermag, wobei die Quantenausbeute des fluoreszierenden Systems klein ist und sich in der Nähe der Inselschicht stark erhöht.

Ein erfindungsgemäßer Sensor besteht somit im Wesentlichen aus
1. einer Schicht aus einer Mehrzahl von nanometrischen Partikeln (=Inseln) aus elektrisch leitendem Material, insbesondere Metall, auf der Oberfläche eines Substrates
2. einer biorekognitiven Schicht auf der genannten Inselschicht unter Zwischenlage einer Spacerschicht
3. mit schwachen oder mäßigen Fluorophoren markierten biorekognitiven Molekülen, wobei der Durchmesser der Inseln kleiner ist als die Wellenlänge des für die Betrachtung bzw. Auswertung verwendeten Lichtes.

Ein erfindungsgemäßes Verfahren zur Messung der Konzentration zumindest eines Analyten in einer Probe zeichnet sich somit dadurch aus,
a) dass die Probe mit einer biorekognitiven Schicht eines Sensors in Kontakt gebracht wird, welche mittels einer Spacerschicht auf zumindest einer Inselschicht mit Inseln aus elektrisch leitendem Material angeordnet ist,
b) dass die Probe mit einem analytspezifischen, fluoreszierenden System mit kleiner Quantenausbeute kontaktiert wird,
c) dass eine Bindung des analytspezifischen, fluoreszierenden Systems an den Analyten sowie die Bindung des Analyten an die biorekognitive Schicht ermöglicht wird, wobei sich die Quantenausbeute des analytspezifischen, fluoreszierenden Systems in der Nähe der Inselschicht stark erhöht,
d) dass eine zur Anregung des analytspezifischen, fluoreszierenden Systems geeignete Anregungsstrahlung in die zumindest eine Inselschicht eingestrahlt wird, sowie
e) dass die vom gebundenen analytspezifischen, fluoreszierenden System emittierte Fluoreszenzstrahlung, als Maß für die Analytkonzentration gemessen wird.

Alternativ zu Punkt c) kann auch eine Bindung des analytspezifischen, fluoreszierenden Sytems und des zu messenden Analyten an die biorekognitive Schicht ermöglicht werden, wobei sich die Quantenausbeute des analytspezifischen, fluoreszierenden Systems in der Nähe der Inselschicht stark erhöht.

Erfindungsgemäß ist vorgesehen, dass das analytspezifische, fluoreszierende System ein fluoreszenzmarkiertes biorekognitives Molekül ist, welches den Analyten zu binden vermag bzw. dass das analytspezifische fluoreszierende System ein fluoreszenzmarkiertes Analytanalogon ist, welches die biorekognitive Schicht zu binden vermag. Bei einem derartigen Sensor wird die Eigenschaft der biorekognitiven Schicht über der Inselschicht ausgenutzt, um entweder:
- nach dem Kontakt mit dem Analyten diesen und ein daran gebundenes, einen Fluoreszenzlabel tragendes, analyterkennendes Molekül als "molekularen Sandwich" zu binden oder
- nach kompetitiver Verdrängung eines, einen Fluoreszenzlabel tragenden Analytanalogon durch den Analyten jenen zu binden.

Eine derartige biorekognitive Bindung führt bei dem erfindungsgemäßen optochemischen Sensor zu einer Veränderung der optischen Eigenschaften, insbesondere zu einer starken Steigerung der Quantenausbeute (Fluoreszenzverstärkung) von Fluorophoren im Bereich 0 bis 20 nm über der Inselschicht.

In konventionellen Fluoreszenzsensoren müssen Fluorophore mit hoher Quantenausbeute eingesetzt werden, um die nötige Empfindlichkeit des Sensors zu gewährleisten. Dabei bewirken jedoch gelöste fluoreszierende Moleküle in der Umgebung des Sensors einen starken, die Empfindlichkeit des Messsystems begrenzenden, Signalhintergrund. Daher muss, um ein gutes Signal zu Rauschverhältnis zu erhalten, der Überschuss an gelöstem Fluorophor vor der Messung vom Sensor entfernt werden. Erfindungsgemäß zeigt der neue Typ des optochemischen Fluoreszenzsensors diesen Nachteil nicht, da die gelösten Moleküle eine sehr geringe Eigenfluoreszenz aufweisen und deren Fluoreszenz erst nach der Bindung an der biorekognitiven Schicht stark erhöht wird. Dadurch wird es - im Gegensatz zu koventionellen Fluoreszenzsensoren - erst möglich, ohne Abtrennung der Analytlösung sofort die biorekognitive Bindung selektiv an der Oberfläche des Sensors zu messen.

Es kann somit mit relativ kurzer Ansprechzeit eine Änderung der optischen Eigenschaften, z. B. der Fluoreszenzintensität oder des Fluoreszenzspektrums, beobachtet werden. Es hat sich überraschenderweise gezeigt, dass bei einer derartigen Struktur die charakteristischen Messeffekte nur im Bereich der energetischen Kopplung (weniger als etwa 20 nm Abstand) der optisch aktiven Moleküle (z. B. Fluorophore) mit der Inselschicht auftreten. Die Ansprechzeit des Sensors ist wie bei allen Sensoren durch die Diffusionszeit des zu messenden Stoffes bis zum Sensormaterial bestimmt, und durch die erfindungsgemäße Ausgestaltung mit überaus dünnen Schichten kann ein entsprechend kurzer Diffusionsweg vorgegeben werden. Mit konventionellen interferometrischen oder Surface Plasmon Resonance Methoden lassen sich aber geringfügige chemische Änderungen in dünnen Schichten nur mit großem messtechnischem Aufwand erfassen. Überraschenderweise hat sich nun gezeigt, dass dann, wenn die Inselschicht auf einer transparenten Oberfläche aufgebracht zur Einkopplung des Messstrahls (z. B. Laser oder LED) verwendet wird, der Messaufbau weitaus einfacher und die Sensitivität des Sensors weitaus größer ist.

Metallische Inselfilme mit einem Durchmesser der Inseln kleiner als die Wellenlänge des für die Betrachtung bzw. Auswertung verwendeten Lichtes zeigen eine starke Absorption, und als deren Folge zeigt das beschriebene System ein ausgeprägtes spektrales Reflexionsmaximum. Fluorophore mit einer Emissionswellenlänge im Inselspektrum, welche eine geringe Quantenausbeute aufweisen, erhöhen ihre Quantenausbeute sehr stark, wenn sie in eine dünne Schicht von etwa 20 nm über die Inselschicht gebracht werden.

Vorteilhafterweise können die Inseln der Inselschicht des Sensors aus Gold oder Silber bestehen. Prinzipiell eignen sich auch andere Metalle, wie beispielsweise Aluminium, für die Ausbildung der Inselschicht. Derartige andere Metalle sind jedoch dem chemischen Angriff in höherem Maße ausgesetzt als die erfindungsgemäß bevorzugt eingesetzte Inselschicht aus Gold oder Silber. Gold und Silber zeichnen sich darüber hinaus durch besonders vorteilhafte Absorptionseigenschaften, damit durch starke Erhöhung der Quantenausbeute und folglich durch hohe Analytempfindlichkeit aus. Grundsätzlich gewinnt ein Fluorophor im Kontakt mit der Inselschicht umso mehr an Quantenausbeute, je niedriger seine Quantenausbeute per se ist. In diesem Punkt unterscheidet sich das erfindungsgemäße Verfahren von allen bekannten Fluoreszenzsensoren, bei denen die Analytempfindlichkeit proportional zur Quantenausbeute des jeweils verwendeten Fluorophors ist.

Die Messgeometrie des optischen Biosensors implementiert die Einstrahlung des Anregungslichts von der probenabgewandten, transparenten Seite des Sensors und die Messung der emittierten Fluoreszenzphotonen in maximalem Raumwinkel auf derselben Seite.

Als Lichtquelle können alle thermischen Emitter, sowie Leuchtdioden und Laser (z. B. frequenzverdoppelter Halbleiterlaser), zur Photonenmessung Photomultipler oder Photohalbleiter (schlechtere Nachweisgrenze) eingesetzt werden.

Eine besonders starke Fluoreszenzverstärkung wird dann beobachtet, wenn die Inseln einen Durchmesser aufweisen, welcher wesentlich kleiner als die Wellenlänge des für die Betrachtung bzw. Auswertung verwendeten Lichtes ist und das Absorptionsminimum mit dem Emissionsmaximum des Fluorophors überlappt. Bevorzugt wird hierbei die Ausgestaltung so getroffen, dass die Inseln einen Durchmesser von kleiner 100 nm, vorzugsweise kleiner 60 nm, bei Verwendung von sichtbarem Licht zur Auswertung aufweisen.

Für die biorekognitive Schicht werden bevorzugt Proteine, wie Antikörper, Antigene und Lektine sowie Hormone, DNA und RNA eingesetzt. Derartige biorekognitive Systeme zeichnen sich durch selektive Bindung des Analyten aus, wobei es genügt, einen derartigen Sensor in Kontakt mit einer Lösung zu bringen, deren Konzentration bestimmt werden soll.

Das analytspezifische, fluoreszierende System kann bevorzugt Proteine, wie Antikörper, Antigene und Lektine sowie Hormone, Lipide, DNA und RNA aufweisen, wobei jedoch diese Moleküle mit einem Fluorophor konjugiert sind. Derartige biorekognitive Systeme zeichnen sich ebenfalls durch selektive Bindung des Analyten aus, wobei es genügt, sie in Lösung mit dem Analyten in Kontakt zu bringen.

Bedingt durch die Dünnschicht und damit kurze Ansprechzeit lässt sich die Erhöhung der Fluoreszenzintensität rasch und sicher erkennen. Gleichzeitig lässt sich aufgrund des relativ einfachen Aufbaus des optochemischen Sensors ein hohes Maß an mechanischer Stabilität gewährleisten. Um eine hinreichend rasche Ansprechzeit zu gewährleisten und gleichzeitig auch ausgeprägte Erhöhung der Fluoreszenzintensität des Schichtsystems zu gewährleisten, wird mit Vorteil die Ausbildung so getroffen, dass die Dicke der immobilisierten biorekognitiven Schicht kleiner als 20 nm, insbesondere kleiner als 15 nm gewählt wird. Zur Erhöhung der Fluoreszenzausbeute soll die Dicke jedoch nicht unter 3 bis 5 nm betragen und es sind prinzipiell Schichtdicken von 5 bis 15 nm optimal realisierbar.

Um die erfindungsgemäß gewünschte hohe Absorption bei gleichzeitig guter Permeabilität bzw. Durchlässigkeit für die Diffusion des zu analysierenden Stoffes aufrecht zu erhalten, ist mit Vorteil die Ausbildung so getroffen, dass eine Inselschicht aus z. B. Gold oder Silber, eine Massendicke kleiner 20 nm, vorzugsweise weniger als 15 nm aufweist, wobei mit Vorteil für besonders hohe Empfindlichkeit die Inselschicht eine Absorption zwischen 40 und 60% für die jeweils verwendete Wellenlänge des Lichtes aufweist.

Der erfindungsgemäße optochemische Sensor kann in einfacher Weise so hergestellt werden, dass auf das Substrat der Inselfilm direkt langsam aufgedampft, aufgesputtert oder durch elektronenstrahllithographische Verfahren hergestellt wird. Durch ein derartiges Aufdampfen lassen sich die erfindungemäß geforderten überaus geringen Massendicke und die Ausbildung voneinander getrennter Inseln sicherstellen, welche zu den charakteristischen spektralen Eigenschaften des Systems führen. Alternativ kann so vorgegangen werden, dass der Inselfilm durch Anlagerung von metallischen Partikeln bzw. Inseln an das Substrat erzeugt oder verändert wird, oder dass die Inseln durch Abtragen von überschüssigem Metall auf der Substratschicht erzeugt werden oder deren Zahl oder Größe verändert wird, wodurch die jeweils gewünschte Massendicke exakt eingestellt werden kann.

Um einen optochemischen Sensor aufzubauen, kann mit Vorteil die Herstellung so durchgeführt werden, dass an der Inselschicht nach Aufbringen einer dünnen (kleiner 10 nm) Zwischenschicht oder Spacerschicht eine biorekognitive Schicht immobilisiert wird. Auf diese Weise kann bei einer biorekognitiven Bindung der Analyt in seiner Konzentration unmittelbar in situ erfasst werden, wobei je Analytmolekül zumindest ein Molekül des fluoreszenzmarkierten Systems gebunden ist.

Mit Vorteil kann die Reaktion der biorekognitiven Schicht und des analytspezifischen, fluoreszierenden Systems in einem Schritt am und über dem Sensor vorgenommen werden. Dabei ist es von Vorteil, für das analytspezifische, fluoreszierende System zumindest einen Fluorophor aus der Gruppe der Fuchsine, Erytrosine, Rhodamine oder ähnliche Moleküle, welche mit einem biorekognitiven Molekül vernetzt sind, einzusetzen.

Es können jedoch auch anlaytspezifische Systme aus zwei energetisch gekoppelten Fluorophoren mit guter Quantenausbeute eingesetzt werden, wobei die Quantenausbeute des primären Fluorophors durch den Energietransfer zum zweiten Fluorophor stark verringert wird. Der Energietransfer wird in unmittelbarer Nähe zur Inselschicht zu den Inseln umgeleitet; der erste Fluorophor zeigt dann eine starke Fluoreszenzemission. Der zweite Fluorophor kann auch durch ein anderes quenchendes, nicht fluoreszierendes Molekül ersetzt werden.

Weiters können jedoch auch Fluorophore mit hoher Quantenausbeute eingesetzt werden, wobei die Quantenausbeute je Fluorophor durch die Bildung von Molekülclustem durch strahlungslose Desaktivierung verringert wird. Diese strahlungslose Desaktivierung wird in unmittelbarer Nähe zur Inselschicht aufgehoben; der Fluorophor zeigt dann eine starke Fluoreszenzemission.

Wesentlich für die erfindunsgemäße Ausbildung ist somit, wie bereits eingangs erwähnt, die überaus dünne Struktur, bei welcher mit wesentlich größeren Ansprechgeschwindigkeiten und kürzeren Ansprechzeiten gerechnet werden kann. Für das charakteristische optische Verhalten ist die anormale Absorption von Inselfilmen, insbesondere eine Absorption im sichtbaren Bereich, wesentliche Voraussetzung, welche damit erklärt wird, dass die Elektronen in Teilchen mit nanometrischem Ausmaß begrenzt beweglich sind, im Gegensatz zu zusammenhängenden Metallschichten, wo metallische Reflexion beobachtet wird.

Prinzipiell kann mit verschiedenen Inselschichtdichten und verschiedenen Fluorophoren jeweils ein Optimum für die Erfassung der Analyten aufgefunden werden. In Versuchen konnte gezeigt werden, dass eine Oberflächenbelegung von z. B. 1/4000 mit fluoreszenzmarkierten Antikörpern für eine optimale Empfindlichkeit des Systems notwendig und auch erreichbar ist.

Alle Veränderungen der Bindung von Fluorophoren können in situ und unmittelbar am Sensor zu einer optischen Anzeige ausgenutzt werden.

Neben mit bifunktionellen Agenzien immobilisierten biorekognitiven Systemen eignen sich auch fotoaktivierbare Agenzien zur Bindung der biorekognitiven Schicht an oder über der Inselschicht.

Schließlich lassen sich die Inseln auch durch Anlagerung von mikrokolloidalen metallischen Partikel erzeugen.

Neben dieser irreversiblen Verwendung des optischen Sensors sind naturgemäß auch optochemische Sensoren von Interesse, welche nach einmaliger Verwendung neuerlich verwendet werden können. Derartige optochemische Sensoren können reversible chemische oder biochemische Bindungsreaktionen ausnutzen, wobei beispielsweise die Goldinselschicht mit einer semipermeablen Membran überzogen ist, welche nur für kleine Analytmoleküle frei durchgängig ist.

Schließlich können mit einem in solcher Weise modifizierten Sensor beispielsweise Glucose, Laktat od. dgl., durch direkte Reaktion des Analyten und eines kompetitierenden fluoreszierenden Analytanalogons mit dem biorekogniven System gemessen werden.

Die Erfindung wird im folgenden anhand von schematischen Zeichnungen näher erläutert.

Es zeigen:
- Fig. 1: einen erfindungsgemäßen Sensor in schematischer Darstellung sowie die
- Fig. 2: bis 4 Ausführungsvarianten des erfindungsgemäßen Sensors.

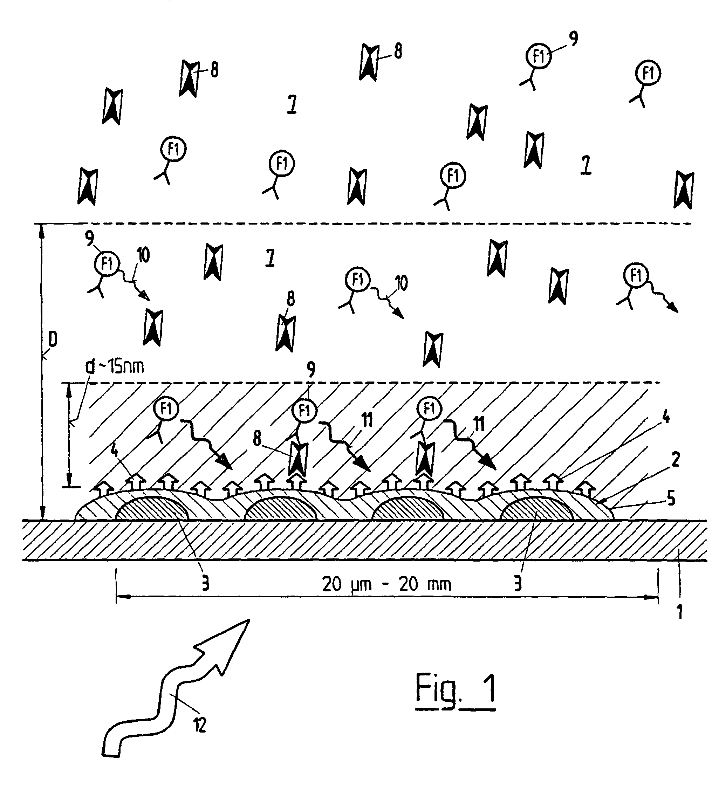

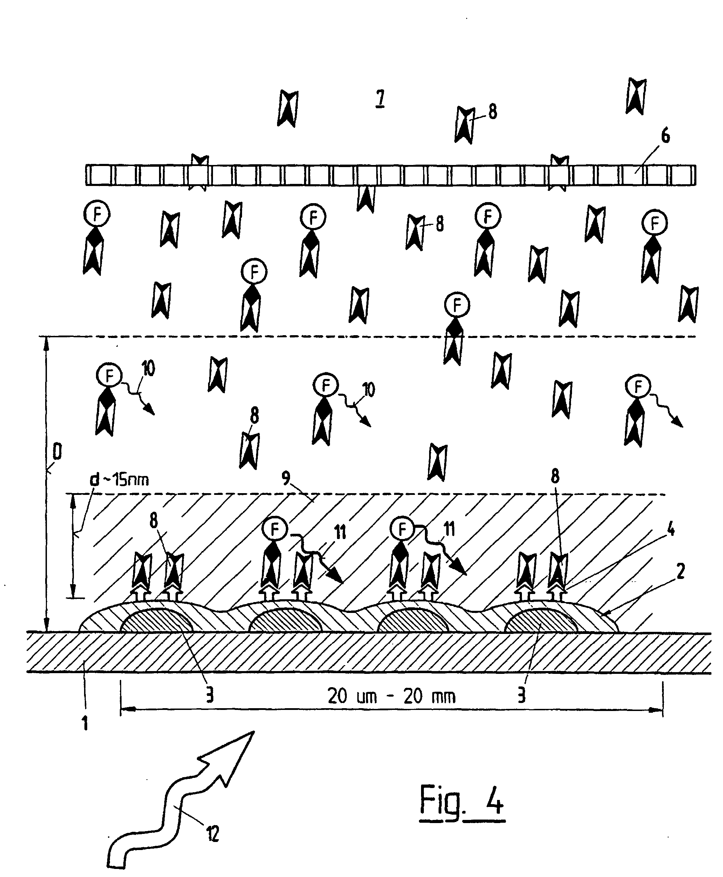

Fig. 1 zeigt einen optochemischen Fluoreszenzsensor mit einem Substrat 1, auf welchen eine Inselschicht 2, bestehend aus einer Vielzahl elektrisch leitender Inseln 3, aufgebracht ist. Die Inseln 3 bestehen vorzugsweise aus Gold oder Silber und weisen einen Durchmesser kleiner 300 nm auf und können beispielsweise durch Aufdampfen auf das Substrat hergestellt werden. Eine biorekognitive Schicht 4 ist (wie in Fig. 1 gezeigt), über eine Spacerschicht 5 an die Inselschicht 2 gebunden. Die biorekognitive Schicht kontaktiert entweder direkt oder über eine analytpermeable Membran 6 (siehe Fig. 4) die Probe 7.

Neben dem Analyten 8 weist die Probe auch ein analytspezifisches, fluoreszierendes System 9 auf, welches der Probe 7 vor der eigentlichen Messung zugesetzt wird oder bereits im Sensor vorliegt. In Fig. 1 besteht das analytspezifische, fluoreszierende System 9 aus einem fluoreszenzmarkierten, biorekognitiven Molekül, welches ursprünglich mit geringer Quantenausbeute, beispielsweise kleiner 30%, fluoresziert (siehe Pfeile 10). Das biorekognitive Molekül mit dem Fluorophor F₁ ist in der Lage, an den Analyten 8 anzukoppeln, welcher seinerseits durch die biorekognitive Schicht 4 auf der Inselschicht 2 gebunden werden kann. In der Nähe der Inselschicht 2, innerhalb einer Distanz d von ca. 15 nm (Bereich der Fluorophor/Insel-Kopplung) erhöht sich die Quantenausbeute des analytspezifischen, fluoreszierenden Systems 9 stark. Die Erhöhung der Fluoreszenzstrahlung (siehe Pfeil 11) ist eine Funktion der Konzentration des Analyten 8. Mit der Distanz D wird die Eindringtiefe der evaneszenten Welle der Anregungsstrahlung 12 dargestellt.

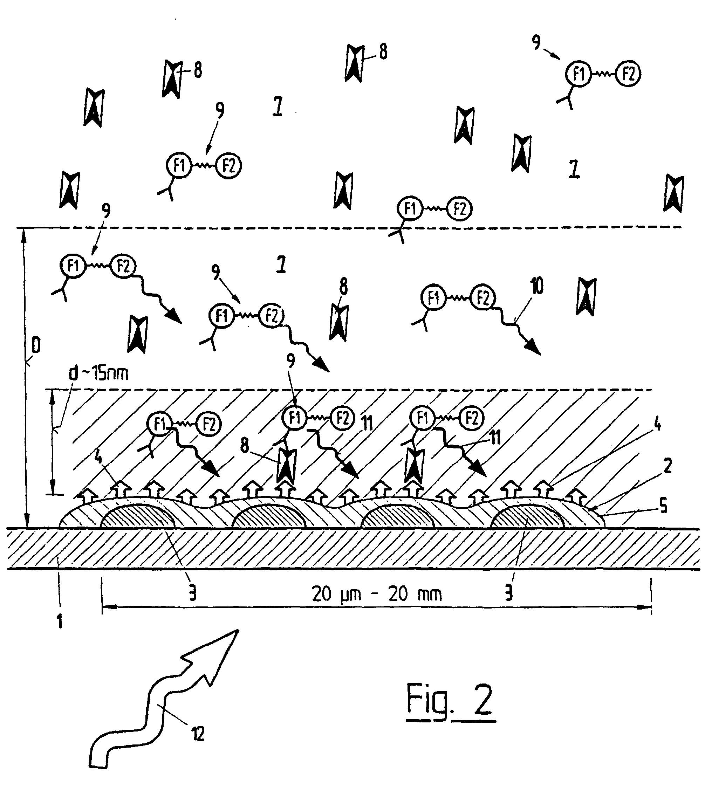

Die Fig. 2 bis 4 zeigen im wesentlichen dieselbe Grundstruktur des Fluoreszenzsensors nach Fig. 1, wobei beispielsweise in Fig. 2 das analytspezifische fluoreszierende System 9 aus zwei gekoppelten Fluorophoren F₁ und F₂ besteht. Die Quantenausbeute des ersten Fluorophors F₁ wird durch Energietransferprozesse zum zweiten Fluorophor F₂ gequencht. In der Nähe der Inselschicht 2 wird die Kopplung zwischen den zwei Fluorophoren aufgehoben, sodass die Intensität der Fluoreszenzstrahlung des Fluorophors F₁ stark ansteigt (siehe Pfeil 11). F₂ kann auch ein nicht fluoreszierendes Molekül sein, welches die Fluoreszenzstrahlung von F₁ quencht.

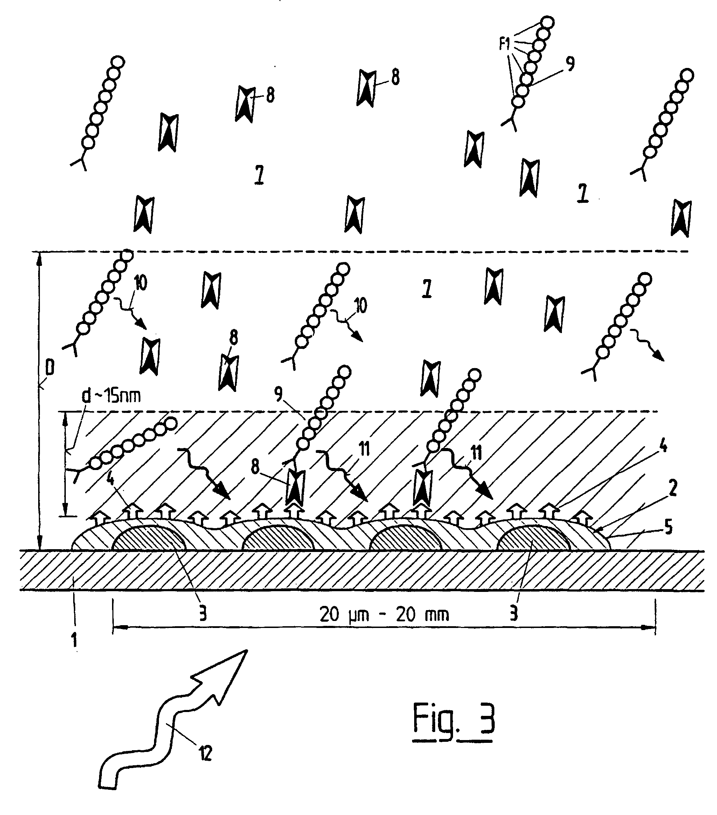

In Fig. 3 besteht das analytspezifische, fluoreszierende System 9 aus einer Vielzahl von Fluorophoren F₁, welche eine räumliche Dichte aufweisen, die zu starker Eigenquenchung führt. Auch hier wird die Eigenquenchung innerhalb der Distanz d (Bereich der Fluorophor/Insel-Kupplung) aufgehoben, sodass die Intensität der Fluoreszenzstrahlung ansteigt.

Schließlich zeigt Fig. 4 ein Beispiel, bei welchem das analytspezifische, fluoreszierende System 9 aus einem fluoreszenzmarkierten Analytanalogon besteht, welches mit dem Analyten 8 eine kompetitive Bindung an der biorekognitiven Schicht 4 eingehen kann.

## Patentansprüche

1. Optochemischer Fluoreszenzsensor zur Messung der Konzentration zumindest eines Analyten (8) in einer Probe, mit zumindest einer auf ein Substrat (1) aufgetragenen Inselschicht (2), deren Inseln (3) aus elektrisch leitendem Material bestehen und einen Durchmesser kleiner 300 nm aufweisen, **dadurch gekennzeichnet, dass** der Sensor eine biorekognitive Schicht (4), bestehend aus Proteinen, Lipiden, Nukleinsäuren oder artifiziellen Liganden aufweist, welche mittels einer Spacerschicht (5) an der Inselschicht (2) angeordnet ist, dass ein der Probe zusetzbares oder im Sensor vorliegendes analytspezifisches, fluoreszierendes System (9) vorgesehen ist, dass die biorekognitive Schicht (4) den zu messenden Analyten (8) direkt oder mittels analytbindender Moleküle zu binden vermag, wobei die Quantenausbeute des fluoreszierenden Systems (9) klein ist und sich in der Nähe der Inselschicht (2) stark erhöht.

2. Sensor nach Anspruch 1, **dadurch gekennzeichnet, dass** das analytspezifische, fluoreszierende System (9) ein fluoreszenzmarkiertes, biorekognitives Molekül ist, welches den Analyten (8) zu binden vermag.

3. Sensor nach Anspruch 1, **dadurch gekennzeichnet, dass** das analytspezifische, fluoreszierende System (9) ein fluoreszenzmarkiertes Analytanalogon ist, welches die biorekognitive Schicht (4) zu binden vermag.

4. Sensor nach Anspruch 2 oder 3, **dadurch gekennzeichnet, dass** der Fluorophor des analytspezifischen, fluoreszierenden Systems (9) eine Quantenausbeute kleiner 30% aufweist.

5. Sensor nach Anspruch 2 oder 3, **dadurch gekennzeichnet, dass** das analytspezifische, fluoreszierende System (9) zwei gekoppelte Moleküle aufweist, von welchen zumindest eines ein Fluorophor ist, wobei die räumliche Nähe zum anderen, die Fluoreszenz des Fluorophors quenchenden Molekül, die Quantenausbeute des Fluorophors verringert.

6. Sensor nach Anspruch 2 oder 3, **dadurch gekennzeichnet, dass** die Moleküle des Fluorophors des analytspezifischen, fluoreszierenden Systems (9) eine räumliche Dichte aufweisen, welche zu starker Eigenquenchung führt.

7. Sensor nach einem der Ansprüche 1 bis 6, **dadurch gekennzeichnet, dass** die Inseln (3) der Inselschicht (2) aus Gold oder Silber bestehen.

8. Sensor nach Anspruch 7, **dadurch gekennzeichnet, dass** die Inseln (3) einen Durchmesser kleiner 100 nm, vorzugsweise kleiner 60 nm aufweisen.

9. Sensor nach einem der Ansprüche 1 bis 8, **dadurch gekennzeichnet, dass** die Inselschicht (2) eine Massendicke kleiner 25 nm, vorzugsweise kleiner 15 nm aufweist.

10. Sensor nach einem der Ansprüche 1 bis 3, **dadurch gekennzeichnet, dass** das analytspezifische, fluoreszierende System (9) zumindest einen Fluorophor aus der Gruppe der Fuchsine, Erytrosine oder Rhodamine aufweist.

11. Sensor nach Anspruch 6, **dadurch gekennzeichnet, dass** der Fluorophor ein Fluorescein ist.

12. Sensor nach Anspruch 5, **dadurch gekennzeichnet, dass** der Fluorophor ein Fluorescein oder ein Rhodamin ist.

13. Verfahren zur Messung der Konzentration zumindest eines Analyten In einer Probe, **dadurch gekennzeichnet:**
a) dass die Probe mit einer biorekognitiven Schicht eines Sensors in Kontakt gebracht wird, welche mittels einer Spacerschicht auf zumindest einer Inselschicht mit Inseln aus elektrisch leitendem Material angeordnet ist,
b) dass die Probe mit einem analytspezifischen, fluoreszierenden System mit kleiner Quantenausbeute kontaktiert wird,
c) dass eine Bindung des analytspezifischen, fluoreszierenden Systems an den Analyten sowie die Bindung des Analyten an die biorekognitive Schicht ermöglicht wird, wobei sich die Quantenausbeute des analytspezifischen, fluoreszierenden Systems in der Nähe der Inselschicht stark erhöht,
d) dass eine zur Anregung des analytspezifischen, fluoreszierenden Systems geeignete Anregungsstrahlung in die zumindest eine Inselschicht eingestrahlt wird, sowie
e) dass die vom gebundenen analytspezifischen, fluoreszierenden System emittierte Fluoreszenzstrahlung, als Maß für die Analytkonzentration gemessen wird.

14. Verfahren nach Anspruch 13, **dadurch gekennzeichnet, dass** alternativ zu Punkt c) eine Bindung des analytspezifischen, fluoreszierenden Systems und des zu messenden Analyten an die biorekognitive Schicht ermöglicht wird, wobei sich die Quantenausbeute des analytspezifischen, fluoreszierenden Systems in der Nähe der Inselschicht stark erhöht.

## Claims

1. Optochemical fluorescence sensor for measuring the concentration of at least one analyte (8) in a sample, with at least one island layer (2) applied on a substrate (1), the islands (3) of said island layer (2) consisting of electrically conductive material and having a diameter smaller than 300 nm, **characterized in that** said sensor is provided with a biorecognitive layer (4) consisting of proteins, lipids, nucleic acids, or artificial ligands, which layer (4) is bound to the island layer (2) via a spacer film (5), and that an analyte-specific fluorescent system (9) is provided in the sensor or for addition to the sample, and that the biorecognitive layer (4) is capable of binding the analyte (8) directly or by means of analyte-binding molecules, the quantum yield of the fluorescent system (9) being small and increasing strongly in the vicinity of the island layer (2).

2. Sensor according to claim 1, **characterized in that** the analyte-specific fluorescent system (9) is a fluorescence-marked biorecognitive molecule capable of bonding the analyte (8).

3. Sensor according to claim 1, **characterized in that** the analyte-specific fluorescent system (9) is a fluorescence-marked analyte analogue capable of bonding to the biorecognitive layer (4).

4. Sensor according to claim 2 or 3, **characterized in that** the fluorophore of the analyte-specific fluorescent system (9) has a quantum yield of less than 30%.

5. Sensor according to claim 2 or 3, **characterized in that** the analyte-specific fluorescent system (9) comprises two coupled molecules, at least one of which is a fluorophore whose quantum yield is reduced by spatial vicinity to the other molecule quenching the fluorescence of the fluorophore.

6. Sensor according to claim 2 or 3, **characterized in that** the molecules of the fluorophore of the analyte-specific fluorescent system (9) have a spatial density which leads to strong self-quenching.

7. Sensor according to any of claims 1 to 6, **characterized in that** the islands (3) of the island layer (2) are made of gold or silver.

8. Sensor according to claim 7, **characterized in that** the diameter of the islands (3) is smaller than 100 nm, and preferably smaller than 60 nm.

9. Sensor according to any of claims 1 to 8, **characterized in that** the island layer (2) has a thickness smaller than 25 nm, and preferably smaller than 15 nm.

10. Sensor according to any of claims 1 to 3, **characterized in that** the analyte-specific fluorescent system (9) contains at least one fluorophore from the group of fuchsines, erythrosines, or rhodamines.

11. Sensor according to claim 6, **characterized in that** the fluorophore is a fluorescein.

12. Sensor according to claim 5, **characterized in that** the fluorophore is a fluorescein or rhodamine.

13. A method for measuring the concentration of at least one analyte contained in a sample, **characterized in that**
(a) the sample is brought into contact with a biorecognitive sensor layer, which is deposited via a spacer film on at least one island layer consisting of islands of electrically conductive material,
(b) the sample is contacted with an analyte-specific fluorescent system of low quantum yield,
(c) a bond is established between the analyte-specific fluorescent system and the analyte, and between the analyte and the biorecognitive layer, the quantum yield of the analyte-specific fluorescent system increasing strongly in the vicinity of the island layer,
(d) the at least one island layer is subject to radiation suitable for excitation of the analyte-specific fluorescent system,
(e) the fluorescence radiation emitted by the bound analyte-specific fluorescent system is determined as a measure for analyte concentration.

14. Method according to claim 13, **characterized in that** as an alternative to item (c) both the analyte-specific fluorescent system and the analyte to be measured are bound by the biorecognitive layer, the quantum yield of the analyte-specific fluorescent system increasing strongly in the vicinity of the island layer.

## Revendications

1. Capteur de fluorescence optochimique pour mesurer la concentration d'au moins un analyte (8) dans un échantillon, comportant au moins une couche en forme d'îlots, appliquée sur un substrat (1), et dont les îlots (3) sont formés d'une matière conductrice électrique, et ayant un diamètre inférieur à 300 nm,
**caractérisé en ce que**
le capteur est une couche de reconnaissance biologique (4) formée de protéines, de lipides, d'acides nucléiques et de ligands artificiels, appliquée avec une couche d'espacement (5) sur la couche à îlots (2),
il est prévu un système fluorescent (9) spécifique à l'analyte que l'on ajoute à l'échantillon ou qui existe dans le capteur,
la couche de reconnaissance biologique (4) peut se combiner à l'analyte (8), directement, ou par l'intermédiaire de molécules liant le composant, le rendement quantique du système fluorescent (9) étant faible et augmentant fortement à proximité de la couche à îlots (2).

2. Capteur selon la revendication 1,
**caractérisé en ce que**
le système fluorescent spécifique au composant (9) est une molécule de reconnaissance biologique de marquage de fluorescence qui peut se combiner à l'analyte (8).

3. Capteur selon la revendication 1,
**caractérisé en ce que**
le système fluorescent (9), spécifique à l'analyte, est un analogue de l'analyte à marquage de fluorescence, qui peut se combiner à la couche de reconnaissance biologique (4).

4. Capteur selon l'une quelconque des revendications 2 ou 3,
**caractérisé en ce que**
le fluorophore du système fluorescent (9) spécifique à l'analyte a un rendement quantique inférieur à 30 %.

5. Capteur selon l'une quelconque des revendications 2 ou 3,
**caractérisé en ce que**
le système fluorescent (9) spécifique à l'analyte comporte deux molécules couplées, dont au moins l'une est un fluorophore, et la proximité spatiale par rapport à l'autre molécule qui assure la trempe de la fluorescence du fluorophore diminue le rendement quantique du fluorophore.

6. Capteur selon l'une quelconque des revendications 2 ou 3,
**caractérisé en ce que**
la molécule du fluorophore du système fluorescent (9) spécifique à l'analyte présente une densité spatiale produisant une forte auto trempe.

7. Capteur selon l'une quelconque des revendications 1 à 6,
**caractérisé en ce que**
les îlots (3) de la couche à îlots (2) sont en or ou en argent.

8. Capteur selon la revendication 7,
**caractérisé en ce que**
les îlots (3) ont un diamètre inférieur à 100 nm, de préférence inférieur à 60 nm.

9. Capteur selon l'une quelconque des revendications 1 à 8,
**caractérisé en ce que**
la couche à îlots (2) a une épaisseur de masse inférieure à 25 nm, de préférence inférieure à 15 nm.

10. Capteur selon l'une quelconque des revendications 1 à 3,
**caractérisé en ce que**
le système fluorescent (9) spécifique à l'analyte comporte au moins un fluorophore du groupe de la fuchsine, de l'érytrosine ou de la rhodamine.

11. Capteur selon la revendication 6,
**caractérisé en ce que**
le fluorophore est une fluorescéine.

12. Capteur selon la revendication 5,
**caractérisé en ce que**
le fluorophore est une fluorescéine ou une rhodamine.

13. Procédé de mesure de la concentration d'au moins un analyte dans un échantillon,
**caractérisé en ce que**
a) on met l'échantillon en contact avec une couche de reconnaissance biologique d'un capteur, cette couche étant installée avec au moins une couche d'écartement sur au moins une couche à îlots comportant des îlots en matière conductrice électrique,
b) on met en contact l'échantillon avec un système fluorescent spécifique à l'analyte, avec un faible rendement quantique,
c) on permet la combinaison du système fluorescent spécifique à l'analyte sur l'analyte ainsi qu'une liaison de l'analyte sur la couche de reconnaissance biologique, et le rendement quantique du système fluorescent spécifique à l'analyte augmente fortement à proximité de la couche à îlots,
d) on injecte un rayonnement d'excitation propre à exciter le système fluorescent spécifique à l'analyte dans au moins une couche d'îlots et
e) on mesure le rayonnement fluorescent émis par le système fluorescent spécifique à l'analyte lié, comme mesure de la concentration à l'analyte.

14. Procédé selon la revendication 13,
**caractérisé en ce qu'**
en variante du point c), on permet une liaison du système fluorescent spécifique à l'analyte et de l'analyte à mesurer sur la couche de reconnaissance biologique et le rendement quantique du système fluorescent spécifique à l'analyte augmente fortement à proximité de la couche d'îlots.
